# EUROPEAN PATENT APPLICATION

(11) **EP 4 803 479 A1**
(43) Date of publication of application: **09.09.2026**
(21) Application number: 26162183.3
(22) Date of filing: 04.03.2026
(51) Int. Cl.: B82Y 15/00, G01N 21/552, G01N 21/64, G01N 33/483

(54) **PLASMON SUBSTRATE AND APPARATUS**

(30) Priority: 07.03.2025 JP 2025035964
(71) Applicant: Canon Kabushiki Kaisha, Tokyo 146-8501 (JP)
(72) Inventor: MIURA, Kaname, Ohta-ku, Tokyo, 146-8501 (JP); EGUCHI, Akira, Ohta-ku, Tokyo, 146-8501 (JP)
(74) Representative: TBK

(57) **Abstract**

A plasmon substrate (1) is configured to detect a specimen, and includes a substrate layer (11), a protrusion portion (2) including a first conductor portion (22) and protruding from the substrate layer, a linker (4) configured to fix the specimen, and a first dielectric portion (21) covering at least a part of the first conductor portion at a top portion of the protrusion portion.

## Description

### TECHNICAL FIELD

The aspect of the disclosure relates to a plasmon substrate that enhances light emitted from a fine particle such as an extracellular vesicle or from a label immobilized on the fine particle, and an apparatus having the plasmon substrate.

### BACKGROUND

A configuration has recently been disclosed in which a fine particle such as an extracellular vesicle (EV) fixed on a plasmon substrate is excited by an enhanced electric field to enhance light emitted from the fine particle or from a label immobilized on the fine particle (see Japanese PCT Domestic Publication No. 2023-521872). In addition, a method has been disclosed in which an area (hot spot) having a high degree of electric field enhancement is formed in a nanogap between conductors in a plasmon substrate to generate an enhanced electric field (see Japanese Patent No. 7375695).

### SUMMARY

The disclosure in its first aspect provides a plasmon substrate as specified in claims 1 to 18.

The disclosure in its second aspect provides an apparatus as specified in claim 19.

Features of the present disclosure will become apparent from the following description of embodiments with reference to the attached drawings. The following description of embodiments is described by way of example.

### BRIEF DESCRIPTION OF THE DRAWINGS

FIG. 1 is a schematic plan view of a principal part of a plasmon substrate according to this embodiment of the disclosure.
FIG. 2 is a cross-sectional view of the principal part of the plasmon substrate according to this embodiment of the disclosure.
FIGs. 3A, 3B, and 3C are cross-sectional views of the principal part of the plasmon substrate according to this embodiment of the disclosure.
FIGs. 4A and 4B are a cross-sectional view and a perspective view of the principal part of the plasmon substrate according to this embodiment of the disclosure.
FIGs. 5A, 5B, 5C, 5D, and 5E are cross-sectional views of the principal part of the plasmon substrate according to this embodiment of the disclosure.
FIGs. 6A, 6B, and 6C are cross-sectional views of the principal part of the plasmon substrate according to this embodiment of the disclosure.
FIGs. 7A, 7B, 7C, 7D, and 7E schematically illustrate the electric field intensity distribution of a plasmon substrate and disposition of EVs on the plasmon substrate in a first embodiment.
FIG. 8 schematically illustrates the structure of the plasmon substrate in the first embodiment.
FIG. 9 illustrates a calculation result of the maximum electric field intensity of the structure of the plasmon substrate in the first embodiment.
FIGs. 10A, 10B, and 10C illustrate calculation results of the electric field intensity excited in EVs in the structure of the plasmon substrate in the first embodiment.
FIGs. 11A and 11B illustrate the electric field intensity distribution of a plasmon substrate in a second embodiment.
FIG. 12 schematically illustrates the structure of the plasmon substrate in the second embodiment.
FIG. 13 illustrates a calculation result of the maximum electric field intensity of the structure of the plasmon substrate in the second embodiment.
FIGs. 14A, 14B, and 14C illustrate calculation results of the electric field intensity excited in EVs in the structure of the plasmon substrate in the second embodiment.
FIGs. 15A and 15B schematically illustrate the structure of a plasmon substrate and the electric field intensity distribution of the structure of the plasmon substrate in a third embodiment.
FIGs. 16A, 16B, and 16C illustrate the electric field intensity distribution of the plasmon substrate in the third embodiment.
FIG. 17 schematically illustrates the structure of a plasmon substrate in a fourth embodiment.
FIGs. 18A and 18B schematically illustrate the structure of a plasmon substrate in a fifth embodiment.
FIGs. 19A and 19B schematically illustrate the structure of a plasmon substrate in a sixth embodiment.
FIGs. 20A and 20B schematically illustrate the structure of a plasmon substrate and the electric field intensity distribution of the structure of the plasmon substrate in a seventh embodiment.

### DESCRIPTION OF THE EMBODIMENTS

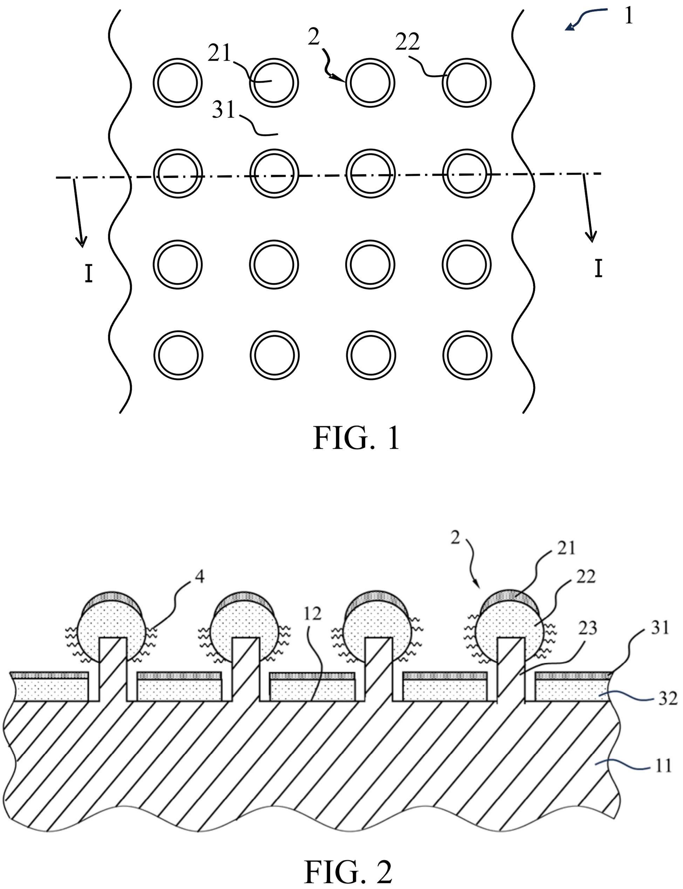

FIG. 1 is a schematic plan view of a principal part of a plasmon substrate 1 according to an embodiment of the disclosure. FIG. 2 is a cross-sectional view of a principal part of the plasmon substrate 1 and is a cross-sectional view along line I-I in FIG. 1.

The plasmon substrate 1 constitutes an examination apparatus together with a light source that irradiates the plasmon substrate 1 with light. The plasmon substrate 1 is configured to utilize surface plasmons and amplify light emitted from a fine particle (sample, analyte, specimen, or test object) having a particle size of several tens of nanometers to several hundreds of nanometers or from a label immobilized on the fine particle, and is used to detect the fine particle. A surface plasmon is a collective vibration of free electrons localized on a metal surface, which causes the localization of an electric field. In this embodiment, an example will be described in which the fine particle is an extracellular vesicle (EV) such as an exosome, a microvesicle, and an apoptotic body, but the disclosure is not limited thereto. EVs secreted from cancer cells contain tumor-derived substances, and cancer can be easily and early found by analyzing substances contained in the EVs.

The plasmon substrate 1 includes a substrate layer 11, protrusion portions 2, a linker 4, and first dielectric portions 21. The protrusion portions 2 are configured to protrude in a direction (protrusion direction) orthogonal to a substrate principal surface 12 of the substrate layer 11 and are randomly or periodically arrayed on the substrate principal surface 12. Each protrusion portion 2 includes a first conductor portion 22 and a pillar 23. The protrusion portions 2 may be periodically arrayed on the substrate principal surface 12 at a pitch of several tens of nanometers to several hundreds of nanometers approximately. The protrusion portions 2 may have cross-sectional areas that are different in the protrusion direction. More specifically, the protrusion portions 2 may be formed such that the cross-sectional area at a first position in the protrusion direction is different from the cross-sectional area at a second position different from the first position in the protrusion direction.

The substrate layer 11 is made of a dielectric such as a resin (an acrylic resin, a fluorine-based resin, an epoxy resin, a silicon-based resin, a urethane resin, PET, a polycarbonate, or an inorganic-organic hybrid material) or glass. Alternatively, the substrate layer 11 may have layers formed of a plurality of conductors or dielectrics such as gold, silver, copper, nickel, aluminum, platinum, or alloys thereof. The substrate layer 11 may have a structure in which a lower layer is a conductor having high reflectance, such as gold or silver, and a transparent dielectric layer is formed thereon.

The pillars 23 are formed on the substrate principal surface 12. The pillars 23 are not particularly limited in shape as long as they protrude from the substrate principal surface 12. Examples of the shape of the pillars 23 include a circular cone, a cylinder, a triangular prism, a quadrilateral prism, a polygonal prism, a circular truncated cone, and a combination thereof. The pillars 23 may be made of a dielectric such as a resin or glass or may be formed of a conductor, but may be formed of a dielectric. The pillars 23 may be made of a material having the same composition as that of the substrate layer 11 or a different composition. The pillars 23 may be integrally formed with the substrate layer 11 or may be bonded thereto. The pillars 23 can be integrally formed by a nanoimprinting method when the substrate layer 11 is made of a resin or a low-melting-point glass. The pillars 23 may be formed on the substrate layer 11 by processing such as etching. The pillars 23 may be formed by immersing an alumina sol solution in warm water or by transferring pillars thus formed onto a conductor.

The first conductor portions 22 are formed on the pillars 23. More specifically, the first conductor portions 22 are disposed on the side of top portions of the protrusion portions 2. Plasmon enhancement occurs at exposed portions of the first conductor portions 22. The first conductor portions 22 each have a size of several nanometers to several hundreds of nanometers approximately. The first conductor portions 22 may be formed, for example, by depositing a conductor on the substrate principal surface 12 by evaporation and performing a method such as sputtering. The first conductor portions 22 may be integrally formed of a conductor having the same composition as that of the pillars 23.

A second conductor portion 32 is another conductor portion between the first conductor portions 22 of adjacent pillars 23 or between the pillars 23. For example, the second conductor portions 32 may be formed on the substrate principal surface 12 as illustrated in FIG. 2. The second conductor portions 32 may be in contact with the pillars 23 or may be spaced therefrom. Surfaces of the second conductor portions 32, which are not in contact with the substrate principal surface 12, may be formed out of contact with the protrusion portions 2. The second conductor portions 32 each have a thickness of several nanometers to several hundreds of nanometers approximately. The second conductor portions 32 may be formed, for example, by depositing a conductor on the substrate principal surface 12 by evaporation and performing a method such as sputtering.

Each first dielectric portion 21 is formed so as to cover a top portion of the first conductor portion 22 (highest position of the first conductor portion 22 in a direction orthogonal to the substrate principal surface 12). The first dielectric portion 21 constitutes the top portion of the protrusion portion 2. A top portion means the highest point. For example, the top portion of the protrusion portion 2 means the highest point of the protrusion portion 2. In a case where there are a plurality of highest points, a point closest to the center of the protrusion portion 2 when the plasmon substrate 1 is viewed from directly above in a direction in which the protrusion portions 2 are visible is defined as a top portion. The first dielectric portion 21 is formed so as to expose at least part of a side portion of the first conductor portion 22. The first dielectric portion 21 has a thickness of several nanometers to several hundreds of nanometers approximately. The first dielectric portion 21 may be formed, for example, by depositing a dielectric such as a resin, silicon, or SiO₂ on the substrate principal surface 12 by a method such as evaporation. In this case, a second dielectric portion 31 may be formed on the second conductor portion 32.

Disposition of the linker 4 can be controlled by the first dielectric portions 21 and the second dielectric portions 31. For example, in a case where the linker 4 that selectively binds to metal ions is used, the linker 4 can be selectively disposed on the exposed portions of the first conductor portions 22 as illustrated in FIG. 2. The first dielectric portions 21 and the second dielectric portions 31 have a function to suppress metal quenching.

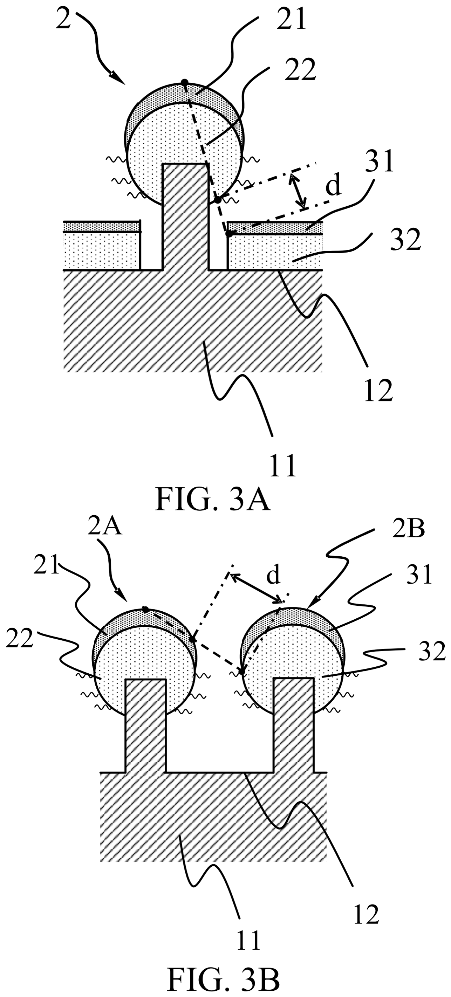

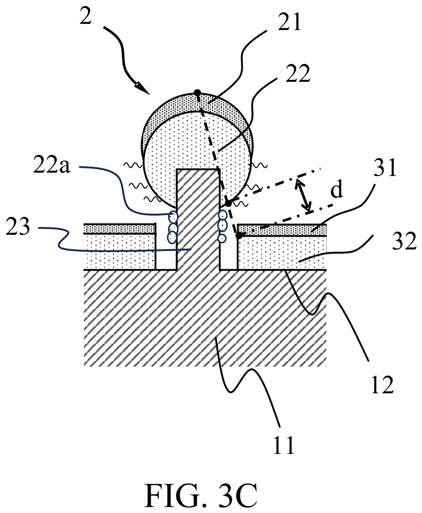

FIGs. 3A, 3B, and 3C are cross-sectional views of a principal part of the plasmon substrate 1. The second conductor portions 32 are adjacent to the protrusion portions 2. More specifically, the second conductor portions 32 are conductors other than the protrusion portions 2 and positioned at the shortest distance from the highest points (position most protruding from the substrate principal surface 12 in the direction orthogonal to the substrate principal surface 12) of the protrusion portions 2. In FIG. 3A, a conductor positioned at the shortest distance from the highest point of a protrusion portion 2 is a conductor layer formed on the substrate principal surface 12, and this conductor layer serves as a second conductor portion 32. In FIG. 3B, two protrusion portions 2 are adjacent to each other, a conductor positioned at the shortest distance from the highest point of one of the protrusion portions 2 is the first conductor portion 22 of the other protrusion portion 2, and this first conductor portion 22 serves as a second conductor portion 32. In this case, the one protrusion portion 2 will be referred to as a first protrusion portion 2A, and the other protrusion portion 2 will be referred to as a second protrusion portion 2B. The first dielectric portion 21 of the second protrusion portion 2B will be referred to as the second dielectric portion 31. The first conductor portion 22 and the first dielectric portion 21 of the first protrusion portion 2A are interchangeable with the second conductor portion 32 and the second dielectric portion 31 of the second protrusion portion 2B. In FIG. 3C, conductor particles 22a in an aggregated state are attached around the pillar 23. In FIG. 3C, the second conductor portion 32 is a conductor layer formed on the substrate principal surface 12, as in FIG. 3A. The second conductor portion 32 forms a hot spot at a position facing the first conductor portion 22.

Since a hot spot is generated at a local place in a plasmon substrate, a fine particle may be disposed at the hot spot in order to excite the fine particle with a strong electric field enhanced at a hot spot.

The distance between the first conductor portion 22 and the second conductor portion 32 can be designed to amplify light emitted from an EV or a label immobilized on the EV. This distance is a distance between a point at which a straight line drawn from the highest position (position farthest from the substrate layer 11) of the protrusion portion 2 in the protrusion direction intersects the second conductor portion 32 in the shortest distance and a point at which the straight line intersects the boundary of the protrusion portion 2, and the distance corresponds to distance d in FIGs. 3A, 3B, and 3C. As an example, the distance d has a size of several nanometers to several hundreds of nanometers approximately. The distance d may have a size of 30 nm to 350 nm or a size of 30 nm to 130 nm. With the distance d designed in this manner, a hot spot is formed between the first conductor portion 22 and the second conductor portion 32. In a case where the EV enters the hot spot, the EV can be excited with an enhanced strong electric field. In other words, light emitted from the EV or a label immobilized on the EV can be enhanced. The EV needs to be disposed near the hot spot to enter the hot spot.

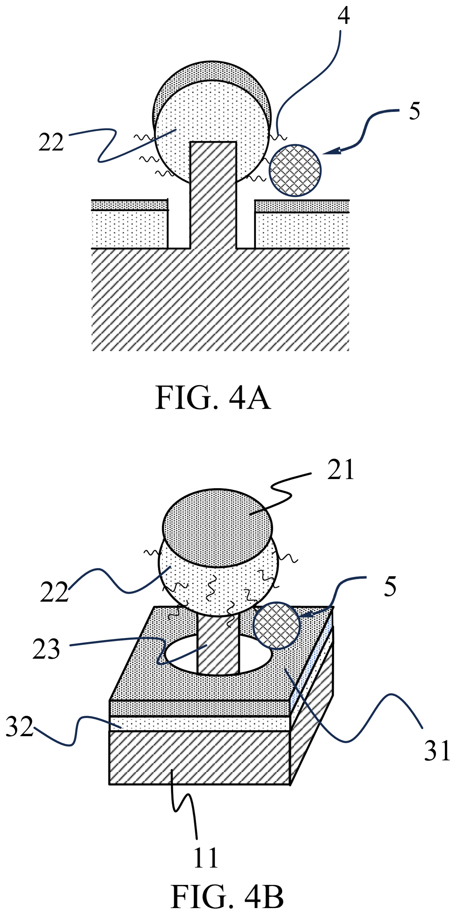

FIGs. 4A and 4B are a cross-sectional view and a perspective view of a principal part of the plasmon substrate 1. The linker 4 selectively binds to an EV 5 and fixes the EV 5 to the plasmon substrate 1. The linker 4 is disposed, for example, on the first conductor portion 22 made of a metal, as illustrated in FIGs. 4A and 4B, and its binding group is bound to metal ions. The binding group of the linker 4 in this case is a chelating group such as an aminopolycarboxylic acid-based chelating group, a hydroxycarboxylic acid-based chelating group, a deferoxamine-derived group, a deferasirox-derived group, a deferiprone-derived group, or a histidine tag. The linker 4 may also be bound to a dielectric other than the first dielectric portion 21, such as the second dielectric portion 31, in order to dispose the EV 5 at a hot spot. The linker 4 may be configured to bind to at least one surface marker on the EV 5 or at least one intravesicular marker in the EV 5. Examples of the markers include tetraspanins, epithelial cell adhesion molecules, and heat shock proteins. In this embodiment, by disposing the linker 4 near the hot spot, the EV 5 is disposed near a gap where the hot spot is formed.

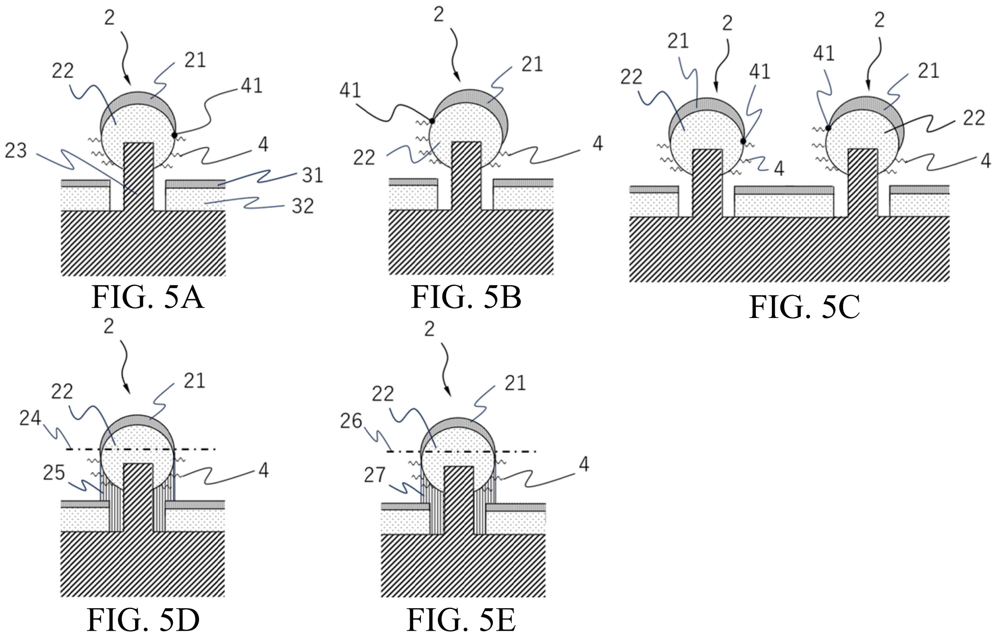

FIGs. 5A, 5B, 5C, 5D, and 5E are cross-sectional views of a principal part of the plasmon substrate 1. As illustrated in FIG. 5A, in the protrusion direction of the protrusion portions 2, the linker 4 is disposed at a position (position opposite to the top portions) lower than a linker disposition maximum height reference point 41 (position closest to the side of the top portions) that is highest on the boundary between the first dielectric portion 21 and the exposed portion of the first conductor portion 22.

As illustrated in FIG. 5B, the boundary between the first dielectric portion 21 and the exposed portion of the first conductor portion 22 is not necessarily disposed at the same height in the protrusion direction of the protrusion portions 2. In this case as well, the linker 4 is disposed at a position lower than the linker disposition maximum height reference point 41.

As illustrated in FIG. 5C, in a case where the linker disposition maximum height reference point 41 is different among a plurality of protrusion portions 2, the linker disposition maximum height reference point 41 is the maximum height at which the linker 4 is disposed in the protrusion portions 2. In this case, positions other than the protrusion portions 2, at which the linker 4 is disposed on the plasmon substrate 1, are lower than the linker disposition maximum height reference point 41 at the highest position in the plurality of protrusion portions 2.

An area in which the linker 4 may be disposed will be described below with reference to FIGs. 5D and 5E. The linker 4 in this embodiment may be disposed in contact with a conductor or a dielectric in a first area 25 (vertically hatched area in FIG. 5D) based on a first section 24. The first section 24 is defined as a section having the largest cross-sectional area among sections of the protrusion portion 2, which are parallel to a surface in contact with the top portions of the protrusion portion 2. The first area 25 is defined as an area included in a columnar body having the first section 24 as a bottom surface and extending in a direction opposite to the top portion of the protrusion portion 2 with respect to the first section 24.

The linker 4 may be disposed in contact with a conductor or a dielectric in a second area based on a second section 26. The second section 26 is defined as a section positioned at the center (including a substantially center) of the protrusion portion 2 among sections of the protrusion portion 2, which are parallel to a surface in contact with the top portion of the protrusion portion 2. A second area 27 is defined as an area included in a columnar body having the second section 26 as a bottom surface and extending in a direction opposite to the top portion of the protrusion portion 2 with respect to the second section. The center of the protrusion portion 2 may be any position between two-tenths to eight-tenths of the height of the protrusion portion 2. The center may be any position between three-tenths to seven-tenths of the height of the protrusion portion 2. The center may be any position between four-tenths to six-tenths of the height of the protrusion portion 2.

By disposing the linker 4 as described above, an EV can be disposed near a hot spot. Thereby, the EV can be excited with an enhanced strong electric field.

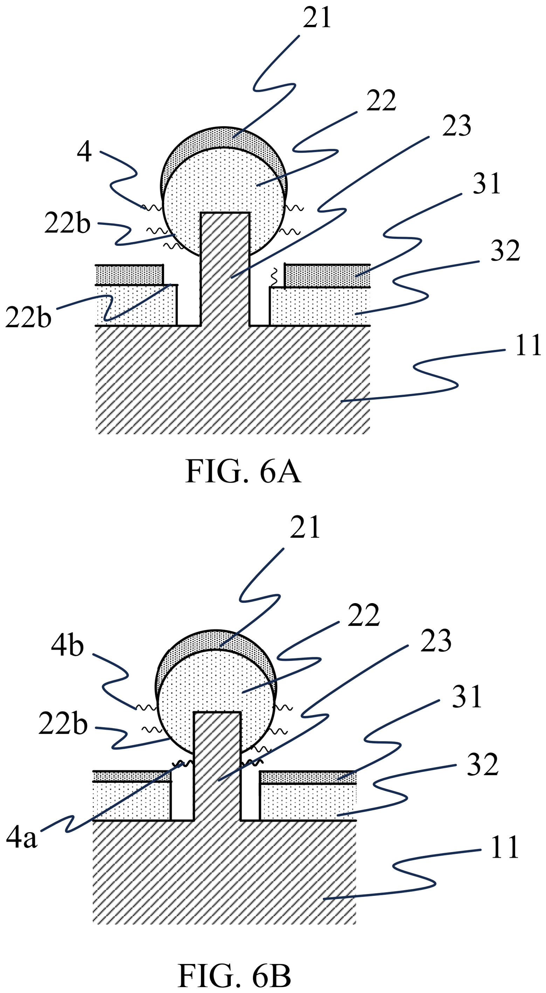

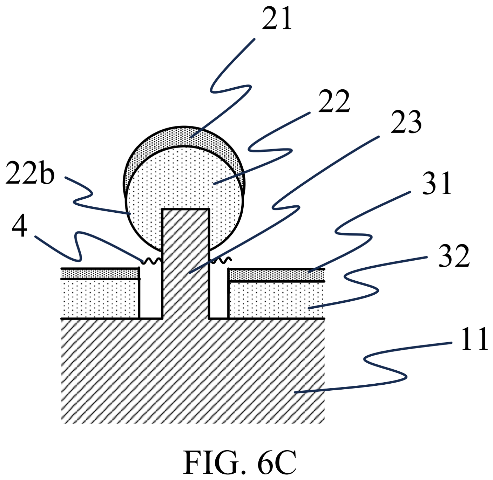

FIGs. 6A, 6B, and 6C are cross-sectional views of a principal part of the plasmon substrate 1. The linker 4 may be disposed at other portions than exposed portions 22b of the first conductor portions 22. As illustrated in FIG. 6A, by partially exposing the second conductor portions 32, the linker 4 can be selectively disposed on the exposed portions 22b. As illustrated in FIG. 6B, a linker 4a may be disposed on the first dielectric portions 21 and the pillars 23. As illustrated in FIG. 6C, the linker 4 may be disposed only on the pillars 23.

Specific configurations in each embodiment will be described below. Light in the embodiment mainly means fluorescence from a labeled dye. However, light in the disclosure is not limited to fluorescent light. Examples below are simulation by a calculator. In each embodiment, electric field intensity distribution is obtained by FDTD calculation. Excitation light is parallel plane waves vertically incident on the substrate principal surface 12. Polarized light is linearly polarized light. In addition, a boundary condition in a direction parallel to the substrate principal surface 12 is a periodic boundary.

### FIRST EMBODIMENT

FIG. 6B schematically illustrates the structure of the plasmon substrate 1 according to this embodiment. In the plasmon substrate 1 in FIG. 6B, the protrusion portions 2 are arrayed in a square lattice (two-dimensional lattice). The period of the protrusion portions 2 is 400 nm. The linker 4a is disposed on the pillars 23, and a linker 4b is disposed on the exposed portions 22b of the first conductor portions 22. In addition, the second conductor portions 32 and the second dielectric portions 31 are formed so as to surround the protrusion portions 2 with a gap interposed therebetween. The first dielectric portions 21 and the second dielectric portions 31 are SiO₂ dielectric films having a thickness of 6 nm. The diameter of each first conductor portion 22 is 160 nm, and the thickness of each second conductor portion 32 is 6 nm. Although the thickness of the second conductor portion 32 is 6 nm in this embodiment, the thickness is not limited to 6 nm as long as at least the side portion of the first conductor portion 22 can be exposed. The thickness of each second conductor portion 32 may be 1 nm to 50 nm. The height of each pillar 23 is 140 nm to 500 nm.

In forming the structure of FIG. 6B, first, the pillars 23 are formed by nanoimprinting on an acrylic resin stacked on a silicon wafer. Next, gold is deposited by evaporation on the substrate layer 11, on which the pillars 23 are formed, to form the first conductor portions 22 and the second conductor portions 32. Then, the linker 4a that binds only to a dielectric is bound to exposed dielectric portions of the pillars 23. The linker 4a is an aminosilane, and a carboxyl group is introduced by oxidizing an acrylic surface, and the aminosilane is bound to the carboxyl group. Then, SiO₂ is deposited to 6 nm by evaporation to form the first dielectric portions 21 and the second dielectric portions 31. In this case, by depositing a thin layer of SiO₂ by evaporation, SiO₂ is formed on the top portions of the first conductor portions 22, and the side portions of the first conductor portions 22 are exposed. Lastly, the linker 4b that binds only to a conductor is bound to the exposed portions 22b to form the structure of FIG. 6B. The linker 4b is polyethylene glycol having a thiol group.

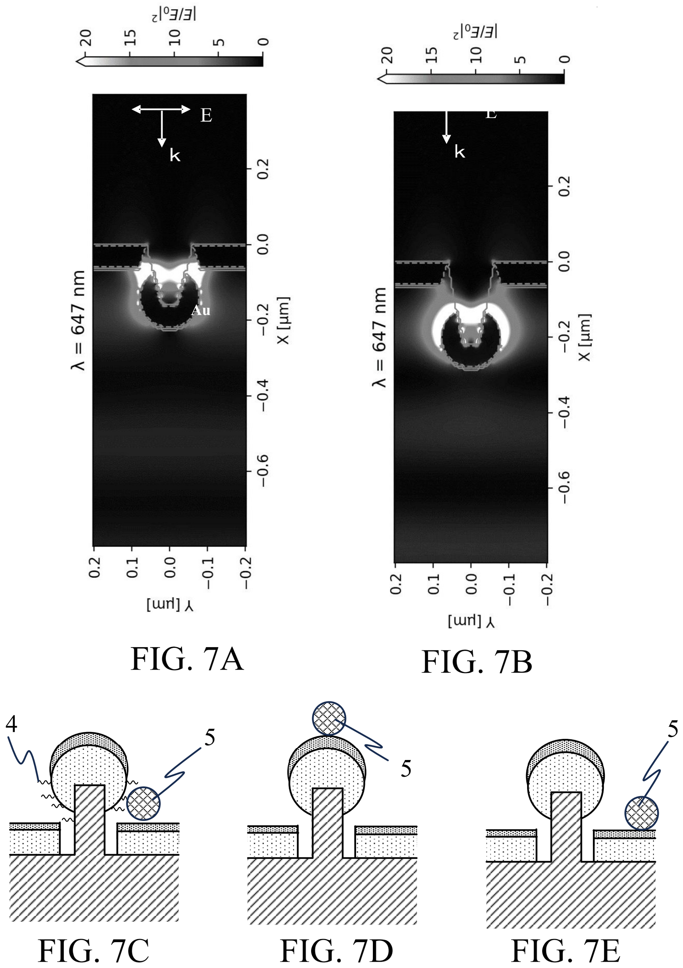

FIGs. 7A and 7B illustrate the electric field intensity distribution of the plasmon substrate 1, which is calculated by simulation. In FIGs. 7A and 7B, the height of the pillar 23 is 160 nm and 220 nm, respectively. A lower portion of the substrate layer 11 is made of silicon, and an upper portion thereof is made of acrylic resin. The first conductor portion 22 and the second conductor portion 32 are made of gold, the first dielectric portion 21 and the second dielectric portion 31 are made of SiO₂. The wavelength of the irradiation light is 647 nm. X and Y directions are defined as illustrated, and a Z direction is defined as the direction of the sheet of FIGs. 7A to 7E in a right-handed coordinate system. Excitation light is polarized in the Y direction, and thus the electric field intensity distribution in FIGs. 7A and 7B is expressed as |sqrt(Ex² + Ez²)/E₀|² by using a y-direction component Ey and an x-direction component Ex of the electric field amplitude, where E₀ is an electric field amplitude calculated for a vacuum space without a structure.

As illustrated in FIGs. 7A and 7B, a hot spot is formed around the protrusion portion 2 and between the first conductor portion 22 and the second conductor portion 32. No electric field enhancement occurs at the top portion of the protrusion portion 2. As a result, an enhanced electric field is potentially excited in the EV 5 when the EV 5 is disposed near the hot spot. On the other hand, even when the EV 5 is disposed at the top portion of the protrusion portion 2, an enhanced electric field may not be able to be excited in the EV 5, for example, in a case where the diameter of the EV 5 is 200 nm or less approximately. Thus, the EV 5 may be disposed near the hot spot rather than the top portion of the protrusion portion 2. The EV 5 may be disposed in a gap between the first conductor portion 22 and the second conductor portion 32 forming the hot spot, and this disposition of the EV 5 is referred to as favorable disposition in the following description. In this embodiment, the first dielectric portion 21 is formed so that the EV 5 is not disposed at the top portion of the protrusion portion 2. Since the first dielectric portion 21 is formed at the top portion of the protrusion portion 2 and a conductor is exposed at a side portion of the protrusion portion 2, the linker 4b that binds only to a metal can be disposed only at the side portion of the protrusion portion 2 as illustrated in FIG. 6B.

As illustrated in FIG. 7C, the EV 5 can be disposed in the favorable disposition by means of the linker 4. On the other hand, when no linker 4 is provided, the EV 5 may be disposed at the top portion of the protrusion portion 2 as illustrated in FIG. 7D or at a position far from the protrusion portion 2 as illustrated in FIG. 7E. The EV 5 is potentially disposed as illustrated in FIGs. 7D and 7E even when the linker 4 is disposed over the entire surface of the plasmon substrate 1.

Results of simulation calculating the electric field intensity excited in a label immobilized around the EV 5 in the favorable disposition of the EV 5 in FIG. 7C and the disposition of FIGs. 7D and 7E will be described below. In this case, the height of the pillar 23 is 220 nm. In FIGs. 7C and 7D, the electric field excited in a label immobilized around the EV 5 having a diameter of 100 nm is five times as strong as that of FIG. 7C. In FIGs. 7C and 7E, the electric field is four times as strong as that of FIG. 7C. That is, limiting the binding position of the linker 4 to near the hot spot on the plasmon substrate 1 and controlling the EV 5 to be disposed in the favorable disposition can excite the EV with a strong electric field enhanced as compared to a case where the EV 5 is disposed at an optional position on the plasmon substrate 1.

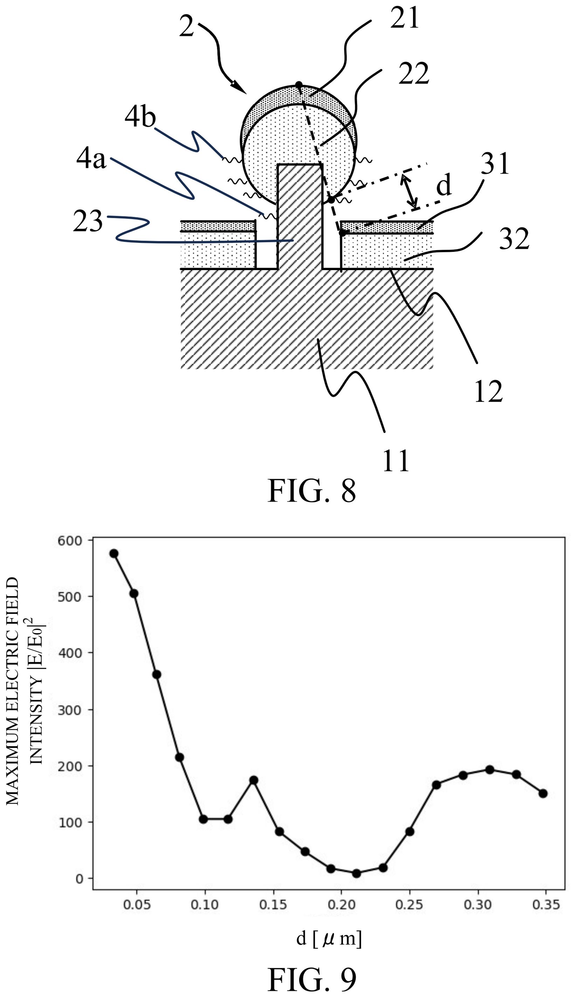

FIG. 8 schematically illustrates the structure of the plasmon substrate 1. In a case where the height of the pillar 23 is changed, a distance d between a point at which a straight line drawn from the highest point of the top portion of the protrusion portion 2 intersects the second conductor portion 32 in the shortest distance and a point at which the straight line intersects the boundary of the protrusion portion 2 changes. When the distance d is changed, the electric field intensity at the hot spot changes.

FIG. 9 illustrates a calculation result of the maximum electric field intensity of the structure of the plasmon substrate 1, i.e., changes in the distance d in FIG. 8 when the height of the pillar 23 is changed, and the maximum electric field intensity in a calculated area. As illustrated in FIG. 9, the maximum electric field intensity tends to increase as the distance d decreases. Such a phenomenon that the maximum electric field intensity increases as the inter-conductor distance decreases is well known. However, when the distance d is decreased too much, the EV 5 cannot be disposed in the gap and cannot be disposed in the favorable disposition. That is, the distance d at which the enhanced electric field can be optimally excited in the EV 5 is potentially different from the distance d that generates the highest maximum electric field intensity.

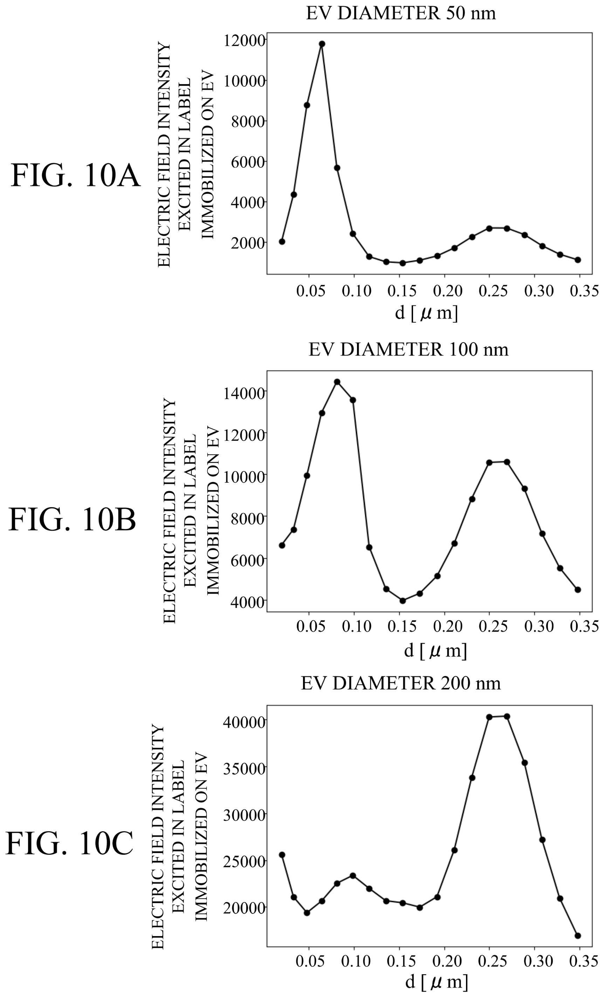

FIGs. 10A, 10B, and 10C illustrate calculation results of the electric field intensity excited in the EV 5 in the structure of the plasmon substrate 1. FIGs. 10A, 10B, and 10C illustrate a relationship between the electric field intensity excited in a label immobilized around the EV 5 and the distance d when the EV 5 having a diameter of 50 nm, 100 nm, and 200 nm, respectively, is fixed to the linker 4. In this case, it is assumed that the EV 5 is fixed through the protrusion portion 2 and the linker 4 and is in contact with the second dielectric portion 31. The linker 4 is disposed at a position lower than the linker disposition maximum height reference point 41. Since a fluorescence dye immobilized on the EV 5 is 10 nm approximately in size, the electric field intensity within 10 nm around the EV 5 is summed. Since incident light is polarized in the Y direction, the electric field intensity around the protrusion portion 2 has anisotropy. Thus, it is assumed that the EV 5 is immobilized at all positions around the protrusion portion 2, the total electric field intensity around the EV 5 is calculated for all circumferential positions of the protrusion portion 2, and its averaged value is shown on the vertical axis in FIGs. 10A, 10B, and 10C. As illustrated in FIGs. 10A, 10B, and 10C, the distance d needs to be properly set to maximize the electric field intensity excited in the label immobilized around the EV 5. A proper distance d is different depending on the diameter of the EV 5, but may be 30 nm to 350 nm. In a case where the diameter of the EV 5 is 50 nm to 100 nm, the distance d may be 30 nm to 130 nm. As illustrated in FIGs. 10A, 10B, and 10C, the electric field intensity excited around the EV 5 is different from the conventionally known phenomenon (result in FIG. 9) that the maximum electric field intensity increases as the inter-conductor distance decreases, and there exists the distance d suitable for the EV 5. In this manner, optimizing (or properly setting) the distance d for the EV 5 and disposing the EV 5 in the favorable disposition by using the linker 4 can excite the EV 5 with a further strong electric field.

The electric field excited in the label immobilized around the EV 5 is mainly described in this embodiment, but the disclosure can also be used to enhance light emitted from the EV 5, such as Raman-scattered light.

### SECOND EMBODIMENT

FIG. 5A schematically illustrates the structure of the plasmon substrate 1 according to this embodiment. This embodiment is different from the first embodiment in that the linker 4 is not disposed on the pillar 23. The difference from the first embodiment in terms of dimensions is that the height of the pillar 23 is 160 nm, and the second conductor portion 32 is disposed with a gap of 50 nm to 150 nm in the radial direction from a central line passing through the protrusion portion 2 in the protrusion direction. In this manner, in this embodiment, the distance between the protrusion portion 2 and the second conductor portion 32 is changed while the height of the pillar 23 is fixed.

In forming the structure of FIG. 5A, first, the pillars 23 are formed by nanoimprinting on an acrylic resin stacked on a silicon wafer. Next, gold is deposited by evaporation on the substrate layer 11, on which the pillars 23 are formed, to form the first conductor portions 22 and the second conductor portions 32. Thereafter, the distance between the second conductor portions 32 and the protrusion portions 2 is set to a predetermined dimension by etching. Then, SiO₂ is deposited to 6 nm by evaporation to form the first dielectric portions 21 and the second dielectric portions 31. Lastly, the linker 4 that binds only to a conductor is bound to the exposed portions of the first conductor portion 22 to form the structure of FIG. 5A.

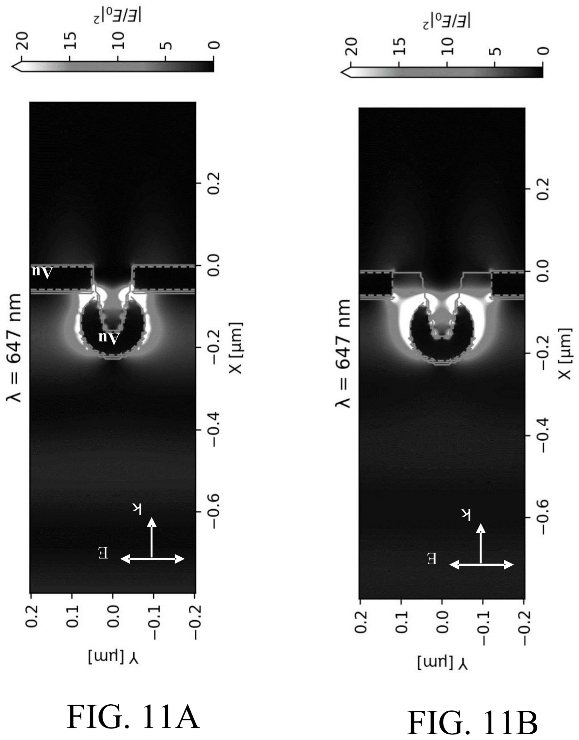

FIGs. 11A and 11B illustrate the electric field intensity distribution of the plasmon substrate 1 according to this embodiment. As illustrated in FIGs. 11A and 11B, a hot spot is formed around the protrusion portion 2 and between the first conductor portion 22 and the second conductor portion 32. No electric field enhancement occurs at the top portion of the protrusion portion 2, and no hot spot is formed other than near the protrusion portion 2. As a result, similarly to the first embodiment, disposing the EV 5 at a favorable position by means of the linker 4 can excite the EV 5 with a strong electric field enhanced at the hot spot.

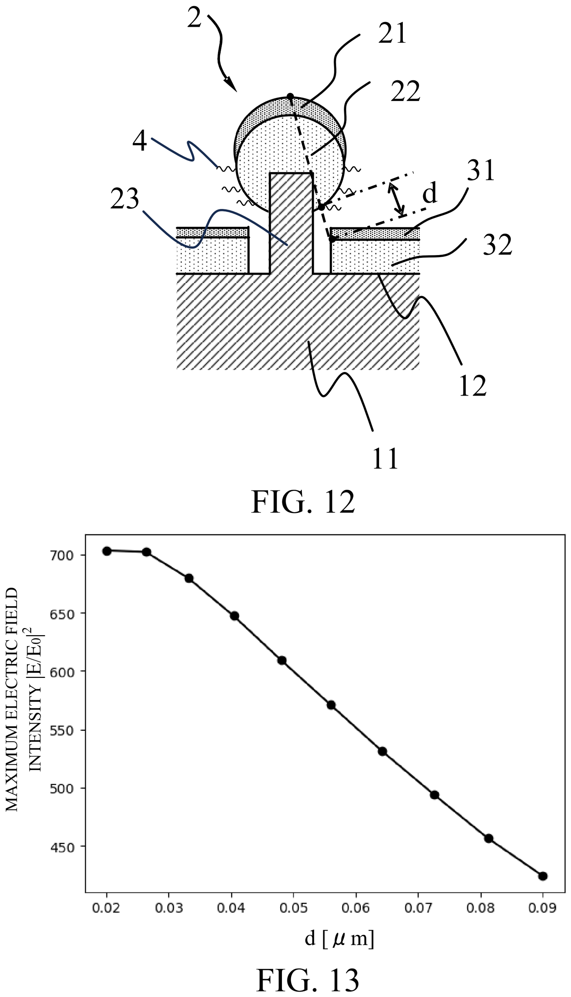

FIG. 12 schematically illustrates the structure of the plasmon substrate 1 according to this embodiment. When the distance between the second conductor portion 32 and the protrusion portion 2 is changed, the distance d between a point at which a straight line drawn from the highest point of the top portion of the protrusion portion 2 intersects the second conductor portion 32 in the shortest distance and a point at which the straight line intersects the boundary of the protrusion portion 2 changes. When the distance d is changed, the electric field intensity at the hot spot changes.

FIG. 13 illustrates a calculation result of the electric field intensity excited in the EV 5 in the structure of the plasmon substrate 1, i.e., changes in the distance d when the distance from the second conductor portion 32 and the second dielectric portion 31 to the pillar 23 is changed, and the maximum electric field intensity in a calculated area. Similarly to the first embodiment, the maximum electric field intensity is defined as the average value of the electric field intensities in the top 100 voxels. As illustrated in FIG. 13, the maximum electric field intensity tends to increase as the distance d decreases.

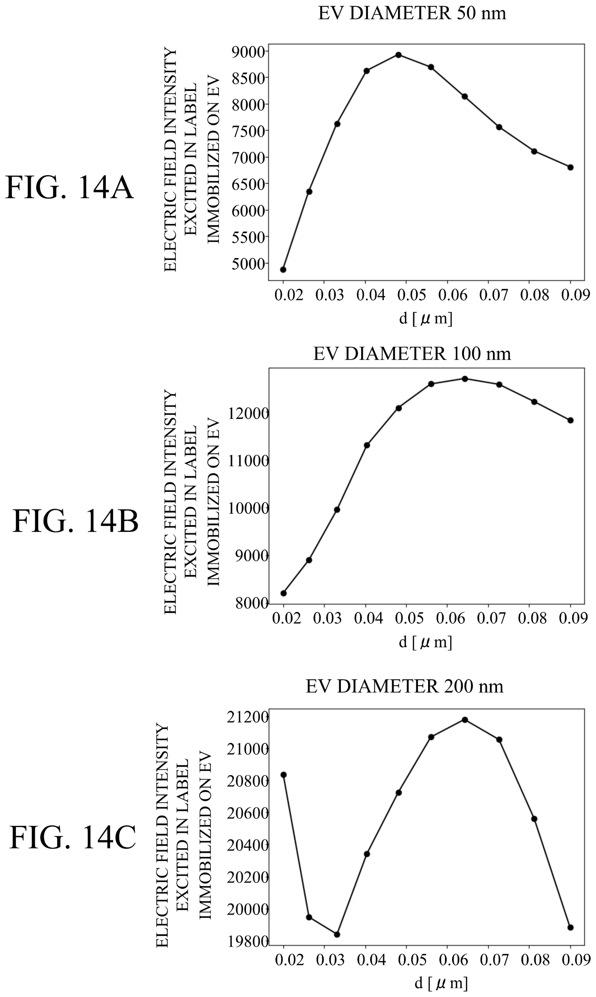

FIGs. 14A, 14B, and 14C illustrate calculation results of the electric field intensity excited in the EV 5 in the structure of the plasmon substrate 1. FIGs. 14A, 14B, and 14C illustrate a relationship between the electric field intensity excited in a label immobilized around the EV 5 and the distance d when the EV 5 having a diameter of 50 nm, 100 nm, and 200 nm, respectively, is fixed to the linker 4. In this case, it is assumed that the EV 5 is fixed through the protrusion portion 2 and the linker 4. As illustrated in FIGs. 14A, 14B, and 14C, the electric field intensity excited in the label immobilized around the EV 5 does not increase as the distance d decreases, and there exists the distance d at which the electric field intensity is maximized. That is, the electric field intensity excited around the EV 5 is different from the conventionally known phenomenon (result in FIG. 13) that the maximum electric field intensity increases as the inter-conductor distance decreases, and there exists the distance d suitable for the EV 5. A proper distance d is different depending on the diameter of the EV 5, but may be 20 nm to 90 nm or 30 nm to 90 nm. Moreover, the proper distance d may be 40 nm to 60 nm when the diameter of the EV 5 is 50 nm approximately, and the proper distance d may be 50 nm to 80 nm when the diameter of the EV 5 is 100 nm approximately or 200 nm approximately.

The electric field excited in the label immobilized around the EV 5 is mainly described in this embodiment, but the disclosure can also be used to enhance light from the EV 5.

### THIRD EMBODIMENT

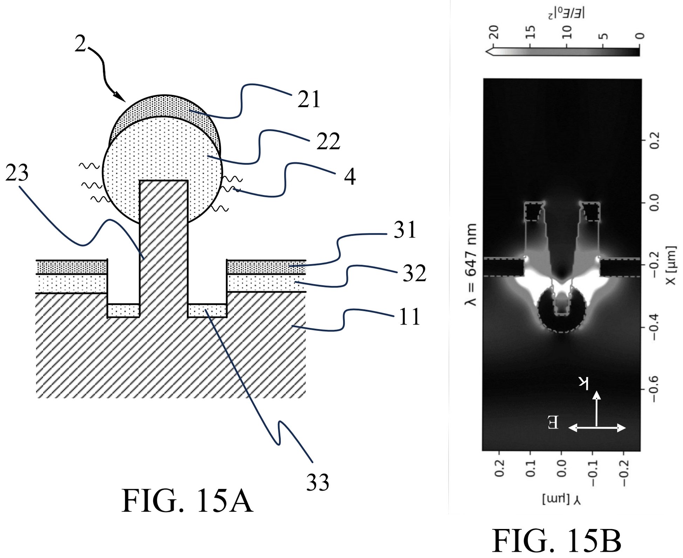

FIGs. 15A and 15B schematically illustrate the structure of the plasmon substrate 1 according to this embodiment, i.e., the electric field intensity distribution of the structure of the plasmon substrate 1.

In forming the structure of FIG. 15A, first, the pillars 23 are formed by nanoimprinting on an acrylic resin stacked on a silicon wafer, concave structures are formed around the pillars 23. Next, gold is deposited by evaporation on the substrate layer 11, on which the pillars 23 are formed, to form the first conductor portions 22, the second conductor portions 32, and third conductor portions 33. Thereby, concave structures are formed in the second conductor portions 32 near the protrusion portions 2. This structure can further increase electric field enhancement in hot spots generated between the first conductor portions 22 and the second conductor portions 32. Then, SiO₂ is deposited to 6 nm by evaporation to form the first dielectric portions 21 and the second dielectric portions 31. Lastly, the linker 4 that binds only to a conductor is bound to the exposed portions of the first conductor portions 22 to form the structure of FIG. 15A.

As illustrated in FIG. 15B, a hot spot is formed around the protrusion portion 2 and between the first conductor portion 22 and the second conductor portion 32. No electric field enhancement occurs at the top portion of the protrusion portion 2. As a result, an enhanced electric field is potentially excited in the EV 5 when the EV 5 is disposed near the hot spot. On the other hand, even when the EV 5 is disposed at the top portion of the protrusion portion 2, an enhanced strong electric field may not be able to be excited in the EV 5, for example, in a case where the diameter of the EV 5 is approximately 200 nm or less. The electric field intensity excited in a label immobilized around the EV 5 having a diameter of 100 nm is compared by simulation between a case where the EV 5 is disposed at a favorable position by means of the linker 4 and a case where the EV 5 is disposed at the top portion of the protrusion portion 2. As a result, the EV 5 disposed at the favorable position can be excited with an electric field that is fifteen times as strong as that of the EV 5 disposed at the top portion of the protrusion portion 2. In this manner, by limiting the binding position of the linker 4 to near the hot spot on the plasmon substrate 1 and disposing the EV 5 in the favorable disposition, it is possible to excite the EV with an enhanced strong electric field.

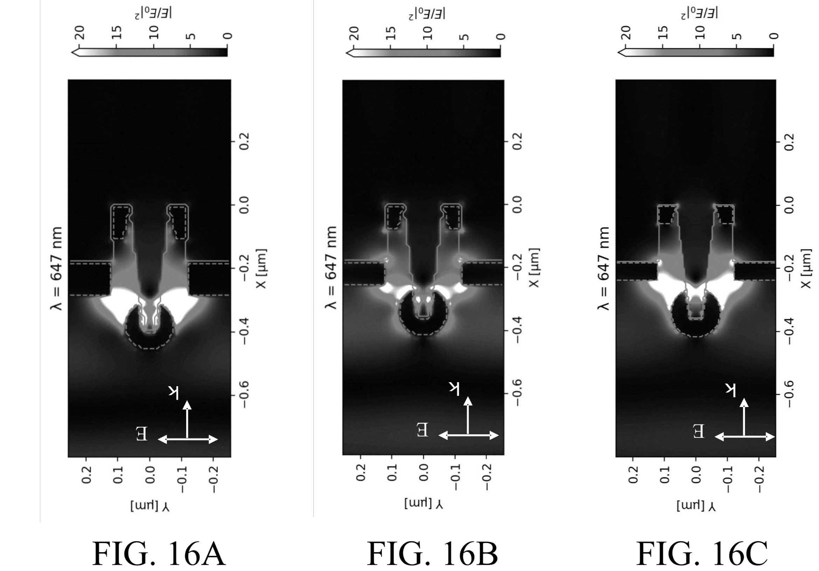

The electric field intensity excited in a label immobilized around the EV 5 having a diameter of 100 nm is compared between a case where the EV 5 is in the favorable disposition in the structure of the first embodiment as illustrated in FIG. 7C and a case where the EV 5 is in the favorable disposition in the structure in the third embodiment. As a result, in the structure in the third embodiment, the EV 5 can be excited with an electric field that is 1.5 times as strong as that in the structure of the first embodiment. That is, forming a concave structure around the protrusion portion 2 and forming the third conductor portion 33 can further increase electric field enhancement at the hot spot. FIGs. 16A, 16B, and 16C illustrate the electric field intensity distribution of the plasmon substrate 1, illustrating calculation examples in cases where the thickness of the second conductor portion 32 is changed from the structure of FIG. 15A. The thickness of the second conductor portion 32 is 100 nm in FIG. 16A, 80 nm in FIG. 16B, and 60 nm in FIG. 16C. The electric field intensity at the hot spot changes as the thickness of the second conductor portion 32 changes. The thickness of the second conductor portion 32 may be 10 nm to 70 nm or 90 nm to 500 nm. The electric field at the hot spot can be further enhanced in accordance with the thickness of the second conductor portion 32.

### FOURTH EMBODIMENT

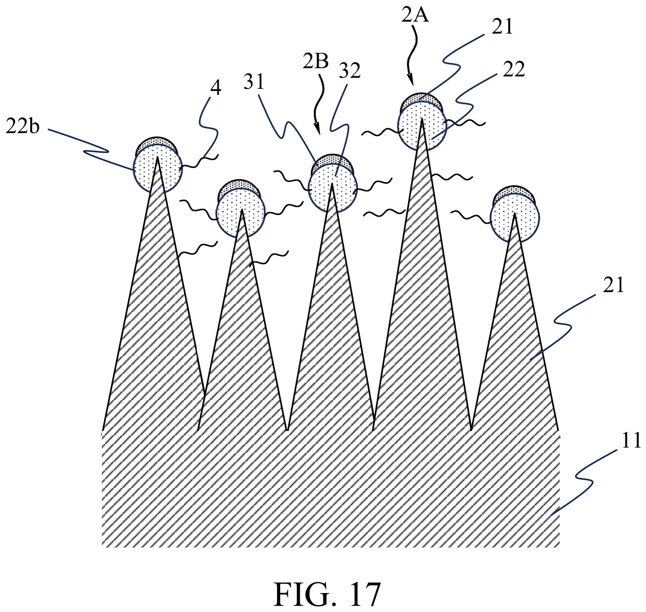

FIG. 17 schematically illustrates the structure of the plasmon substrate 1 according to this embodiment. In the plasmon substrate 1 according to this embodiment, the protrusion portions 2 are formed on and integrated with the substrate layer 11. The substrate layer 11 and the protrusion portions 2 are formed of Ni. The period of the protrusion portions 2 is random but falls within a range of 10 nm to 500 nm. In addition, the height of the protrusion portions 2 is random, and the average of the height falls within a range of 100 nm to 1000 nm. Granular gold bodies are formed at upper portions of the protrusion portions 2, and dielectric films are deposited on upper portions thereof. In this structure, a first conductor portion 22 is the granular gold body at the upper portion of an optional protrusion portion 2, and a first dielectric portion 21 is a dielectric film at the upper portion of the protrusion portion 2. In this case, a second conductor portion 32 is a conductor closest to the top portion of the protrusion portion 2. For example, when the second protrusion from the right in FIG. 17 is referred to as the first protrusion portion 2A, the second conductor portion 32 is a conductor portion of the second protrusion portion 2B, which is a conductor existing at the shortest distance from the highest point of the first protrusion portion 2A. In the plasmon substrate 1 according to this embodiment, a hot spot is formed between the first conductor portion 22 and the second conductor portion 32. The EV 5 can be selectively disposed near the hot spot by disposing the linker 4.

In forming the structure of FIG. 17, first, alumina protrusions are formed by immersing an alumina sol solution in warm water. Next, the alumina protrusions are transferred onto nickel, and alumina is removed by an etching process to form the pillars 23. In this case, residual alumina may remain. Next, gold is deposited on the pillars 23 by evaporation to form the first conductor portions 22, and thereafter, SiO₂ is deposited by evaporation to form the first dielectric portions 21. Lastly, the linker 4 is bound to the exposed portions 22b of the first conductor portions 22 to form the structure of FIG. 17.

### FIFTH EMBODIMENT

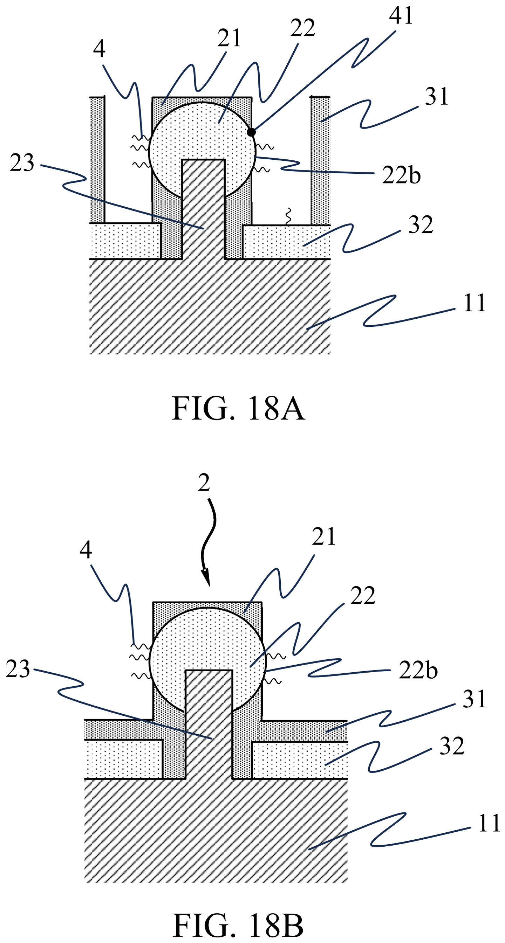

FIGs. 18A and 18B schematically illustrate the structure of the plasmon substrate 1 according to this embodiment. The plasmon substrate 1 according to this embodiment is different from the plasmon substrate 1 in the first to third embodiment in that the first dielectric portions 21 and the second dielectric portions 31 are formed of a dielectric layer having a concave structure. The concave structure of the dielectric layer is a structure for receiving the EV 5. In the structure of FIG. 18A, the dielectric layer is formed on the pillar 23, the first conductor portion 22, and the second conductor portion 32 formed on the substrate layer 11. Then, circular dielectric concave structures are in contact with both ends of the first conductor portion 22, and as a result, the exposed portion 22b is formed. The exposed portion 22b is also formed on the second conductor portion 32. In this case, the pillar 23, the first conductor portion 22, and the first dielectric portion 21 are referred to as a protrusion portion 2. The first dielectric portion 21 is located at a position higher than the linker disposition maximum height reference point 41, and is a dielectric in an upper projection range of the first conductor portion 22. The first dielectric portion 21 functions to prevent the linker 4 from being disposed at the top portion of the protrusion portion 2. The second dielectric portion 31 functions to prevent the linker 4 from being disposed at a position at which no hot spot is formed except for the top portion of the protrusion portion 2. Thus, in this embodiment, the second dielectric portion 31 is a dielectric other than the first dielectric portion 21. The diameter of the concave structure of the dielectric layer is 5 nm to 800 nm and may be 10 nm to 500 nm or 50 nm to 300 nm.

In the structure of FIG. 18A, the linker 4 is disposed on the exposed portion 22b of the first conductor portion 22. The structure of FIG. 18B is a modification of FIG. 18A, and the second conductor portion 32 is covered by the second dielectric portion 31. The thickness of the second dielectric portion 31 is 1 nm to 500 nm so that the upper surface of the second dielectric portion 31 is lower than the height of the pillar 23. In the structure of FIG. 18B, the dielectric concave structure is a structure recessed from the top portion of the protrusion portion 2. Thus, the shape of the concave structure does not need to be circular and is not particularly limited as long as the exposed portion 22b is formed in the first conductor portion 22. For example, a dielectric at a position higher than the upper surface of the second dielectric portion 31 may be located only in the upper projection range of the protrusion portion 2. As other modifications of FIG. 18A, the shape of the concave structure may be polygonal, and the concave structure may be formed in plurality or in a ring shape around the first conductor portion 22.

In the structures of FIGs. 18A and 18B, a hot spot is formed between the first conductor portion 22 and the second conductor portion 32. The EV 5 can be selectively disposed near the hot spot by disposing the linker 4.

In forming the structures of FIGs. 18A and 18B, gold is deposited by evaporation on the substrate layer 11, which includes the pillars 23 formed as in the first embodiment, to form the first conductor portions 22 and the second conductor portions 32. Next, a dielectric is deposited to a position higher than the first conductor portions 22. Thereafter, the first dielectric portions 21 are formed by performing an etching process so as to form the exposed portions 22b. Lastly, the linker 4 is bound to the exposed portions 22b to form the structures of FIGs. 18A and 18B.

### SIXTH EMBODIMENT

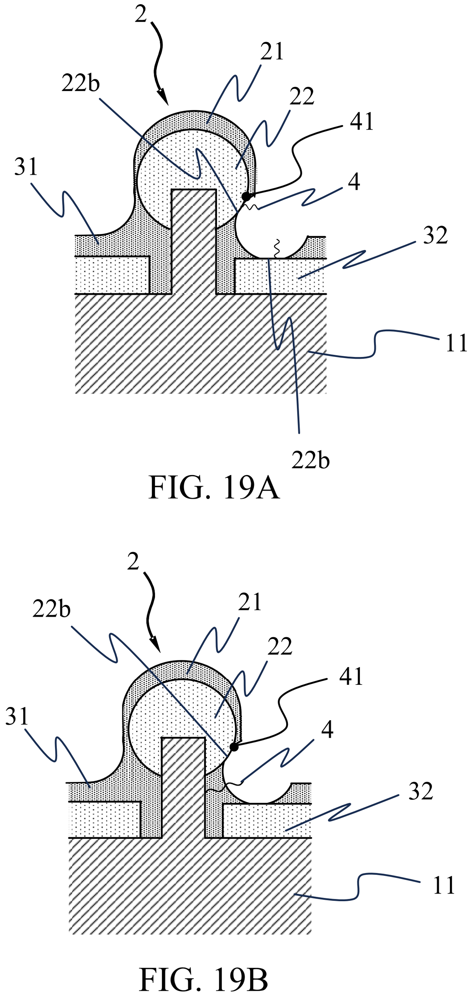

FIGs. 19A and 19B schematically illustrate the structure of the plasmon substrate 1 according to this embodiment. In this embodiment, when a conductor portion formed in the protrusion direction of each pillar 23 and a conductor layer formed on the substrate layer 11 are covered with an integral dielectric, the pillar 23, the conductor portion formed in the protrusion direction of the pillar 23, a dielectric formed at a top portion of the conductor portion are referred to as a protrusion portion 2. The dielectric formed at the top portion of the conductor portion is a dielectric at a position higher than the linker disposition maximum height reference point 41. Thus, as illustrated in FIGs. 19A and 19B, the conductor portion formed in the protrusion direction of the pillar 23 is referred to as a first conductor portion 22, and the conductor layer formed on the substrate layer 11 is referred to as a second conductor portion 32. In the structures of FIGs. 19A and 19B, a hot spot is formed between the first conductor portion 22 and the second conductor portion 32. The EV 5 can be selectively disposed near the hot spot by disposing the linker 4.

In forming the structure of FIG. 19A, first, gold is deposited by evaporation on the substrate layer 11, on which the pillars 23 are formed, to form the first conductor portions 22 and the second conductor portions 32. Next, silica nanobeads with an adjusted concentration are dropped and dried. When dried, the silica nanobeads aggregate on the protrusion portions 2, and thus the silica beads are disposed in contact with the protrusion portions 2 and the second conductor portions 32. Next, a polymer thin film is formed by surface-initiated atom transfer radical polymerization. The substrate on which the silica nanobeads are disposed is put into a beaker, a mixed solution of 2-methacryloyloxyethyl phosphorylcholine, 2,2'-bipyridyl, and CuBr2 together with PBS is added, and then L-ascorbic acid is injected under a nitrogen atmosphere to perform polymerization. Thereby, a polymer thin film in which nano silica beads and a polymer are copolymerized is obtained on the substrate, in which the first conductor portions 22 and the second conductor portions 32 are formed on the substrate layer 11. Next, the silica nanobeads are removed. The silica nanobeads are removed by cleaving the copolymerization of the polymer and silica nanobeads with a tris(2-carboxyethyl)phosphine·HCl (TCEP) aqueous solution and then washing out the silica nanobeads. Lastly, the linker 4 is bound to the exposed portions 22b of the first conductor portions 22.

In forming the structure of FIG. 19B, first, gold is deposited by evaporation on the substrate layer 11, on which dielectric pillars are formed, to form the first conductor portions 22 and the second conductor portions 32. Next, the linker 4 is bound to the dielectric. Then, silica nanobeads modified to bind to the linker 4 are dropped and dried, and a polymer thin film is formed. Lastly, the silica nanobeads are removed to form the structure of FIG. 19B.

### SEVENTH EMBODIMENT

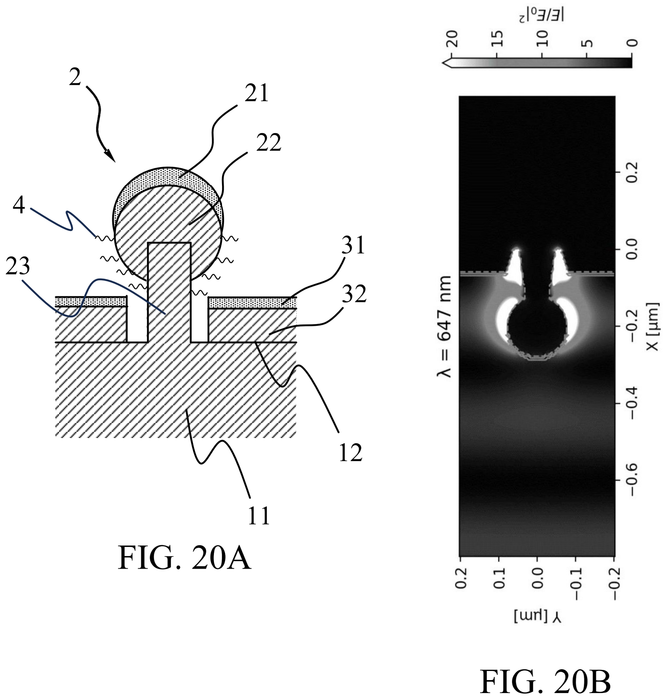

FIG. 20A schematically illustrates the structure of the plasmon substrate 1 according to this embodiment. In this embodiment, the protrusion portions 2 are formed on the substrate layer 11, and the protrusion portions 2 and the substrate layer 11 are integrated. The substrate layer 11 and the protrusion portions 2 are formed of gold. The period of the protrusion portions 2 is 400 nm, and the height thereof is 300 nm. Granular gold bodies are formed at upper portions of the protrusion portions 2, and dielectric films are deposited on the upper portions. In this structure, the first conductor portions 22 are conductors constituting the protrusion portions 2, and the pillars 23 are also the first conductor portions 22. The first dielectric portions 21 are the dielectric films on the upper portions of the protrusion portions 2. In this case, the second conductor portions 32 are the closest conductors that are adjacent to the top portions of the protrusion portions 2, and in this embodiment, are conductors formed on the substrate layer 11. FIG. 20B illustrates the electric field intensity distribution obtained by simulation. As illustrated in FIG. 20B, in the structure according to this embodiment, a hot spot is formed between each first conductor portion 22 and the corresponding second conductor portion 32. The EV 5 can be selectively disposed near the hot spot by disposing the linker 4.

In forming the structure of FIG. 20A, first, a resist is applied on the substrate layer 11 which is a gold flat plate, a pattern is transferred by nanoimprinting, and thereafter, etching is performed to form the pillars 23 on the gold surface. Next, gold is deposited by evaporation on the substrate layer 11 including the formed pillars 23 to form the first conductor portions 22 and the second conductor portions 32. Then, SiO₂ is deposited to 5 nm, and lastly, the linker 4 is bound to the exposed portions of the first conductor portions 22 to form the structure of FIG. 20A.

As a modification according to this embodiment, the first conductor portions 22, the pillars 23, and the substrate layer 11 may be integrally formed of conductors such as silver, copper, nickel, aluminum, platinum, or alloys thereof, or may be formed of different metals, respectively.

While the present disclosure has been described with reference to embodiments, it is to be understood that the present disclosure is not limited to the disclosed embodiments. The scope of the following claims is to be accorded the broadest interpretation so as to encompass all such modifications and equivalent structures and functions.

Each embodiment can provide a plasmon substrate that excites a strong electric field enhanced at a hot spot in a fine particle.

## Claims

1. A plasmon substrate (1) configured to detect a specimen, the plasmon substrate comprising:
a substrate layer (11);
a protrusion portion (2) including a first conductor portion (22) and protruding from the substrate layer;
a linker (4) configured to fix the specimen; and
a first dielectric portion (21) covering at least a part of the first conductor portion at a top portion of the protrusion portion.

2. The plasmon substrate according to claim 1, **characterized in that** the linker is disposed on a side opposite to the top portion with respect to a position closest to the top portion on a boundary between the first dielectric portion and the first conductor portion.

3. The plasmon substrate according to claim 1 or 2, **characterized in that** the protrusion portion is formed such that, in a protrusion direction of the protrusion portion, a cross-sectional area at a first position is different from a cross-sectional area at a second position different from the first position.

4. The plasmon substrate according to claim 3, **characterized in that** the linker is disposed in contact with a conductor or a dielectric in a first area (25) based on a first section (24),
wherein the first section is a section of the protrusion portion, the section having a largest cross-sectional area among sections parallel to a surface in contact with the top portion, and
wherein the first area is an area included in a columnar body having the first section as a bottom surface and extending in a direction opposite to the top portion with respect to the first section.

5. The plasmon substrate according to claim 3, **characterized in that** the linker is disposed in contact with a conductor or a dielectric in a second area (27)based on a second section (26),
wherein the second section is a section of the protrusion portion, the section being positioned at a center of the protrusion portion among sections parallel to a surface in contact with the top portion, and
wherein the second area is an area included in a columnar body having the second section as a bottom surface and extending in a direction opposite to the top portion with respect to the second section.

6. The plasmon substrate according to any one of claims 1 to 5, further comprising a second conductor portion (32) disposed adjacent to the protrusion portion.

7. The plasmon substrate according to claim 6, **characterized in that** the second conductor portion is disposed on a side opposite to the top portion with respect to a position closest to the top portion on a boundary between the first dielectric portion and the first conductor portion.

8. The plasmon substrate according to claim 6, **characterized in that** the second conductor portion has the same structure as that of the first conductor portion.

9. The plasmon substrate according to any one of claims 6 to 8, **characterized in that**, in a protrusion direction of the protrusion portion, a distance between a point at which a straight line drawn from a position farthest from the substrate layer intersects the second conductor portion in a shortest distance and a point at which the straight line intersects a boundary of the protrusion portion is 30 nm to 350 nm.

10. The plasmon substrate according to claim 6, further comprising a second dielectric portion (31) disposed on the second conductor portion.

11. The plasmon substrate according to any one of claims 1 to 10, **characterized in that** the linker selectively binds to the specimen and binds the specimen to the plasmon substrate.

12. The plasmon substrate according to any one of claims 1 to 11, **characterized in that** the linker is disposed only on a conductor.

13. The plasmon substrate according to any one of claims 1 to 12, **characterized in that** the first dielectric portion is a dielectric film.

14. The plasmon substrate according to any one of claims 1 to 12, **characterized in that** the first dielectric portion is a dielectric layer having a concave structure.

15. An examination apparatus comprising:
the plasmon substrate according to any one of claims 1 to 14; and
a light source configured to illuminate the plasmon substrate.
